# FASCICULE DE BREVET EUROPEEN

(11) **EP 4 188 484 B1**
(45) Date de publication et mention de la délivrance du brevet: **18.02.2026**
(21) Numéro de dépôt: 21751527.9
(22) Date de dépôt: 23.07.2021
(51) Int. Cl.: A61M 5/315

(54) **SERINGUE A HAUTE RESISTANCE A L'ARRACHEMENT**
SPRITZE MIT HOHEM AUSZIEHWIDERSTAND
SYRINGE WITH HIGH PULL-OUT RESISTANCE

(30) Priorité: 31.07.2020 FR 2008157
(43) Date de publication de la demande: 07.06.2023
(73) Titulaire: Laboratoires Vivacy, 75116 Paris (FR)
(72) Inventeur: LE BAIL, Noémie, 74350 COPPONEX (FR); GOULINET, Philippe, 74330 LA BALME DE SILLINGY (FR)
(74) Mandataire: INNOV-GROUP
(86) Numéro de dépôt international: PCT/EP2021/070746
(87) Numéro de publication internationale: WO 2022/023220

(56) Documents cités:
- EP-A2- 0 354 368
- WO-A1-03/047668
- WO-A1-2011/023738
- WO-A2-2008/064283
- US-A1- 2011 034 882

## Description

### Domaine technique

L'invention se rapporte au domaine technique des seringues.

L'invention trouve notamment son application dans l'injection de gels viscoélastiques à base d'acide hyaluronique, dans le domaine de l'anti-âge et de l'esthétique médicale.

### État de l'art

Une seringue connue de l'état de la technique comporte :
- un corps de seringue, destiné à recevoir un produit à injecter, et comprenant un butoir ; le butoir présentant une paroi interne, élastiquement déformable, et délimitant une cavité ; le corps de seringue étant destiné à être équipé d'un porte-aiguille ;
- un piston, monté coulissant à l'intérieur du corps de seringue suivant un sens d'injection du produit et suivant un sens opposé, et comprenant une tige présentant une première extrémité et une seconde extrémité opposée ;
- un poussoir, agencé à la première extrémité de la tige ;
- une tête d'encliquetage, agencée à la seconde extrémité de la tige, et encliquetée à l'intérieur de la cavité du butoir ; la tête d'encliquetage présentant une surface d'appui plane venant en appui contre la paroi interne du butoir lorsque le piston coulisse suivant le sens opposé au sens d'injection du produit.

Nous emploierons par la suite le terme « médecin », étant entendu qu'il désigne tout utilisateur de la seringue.

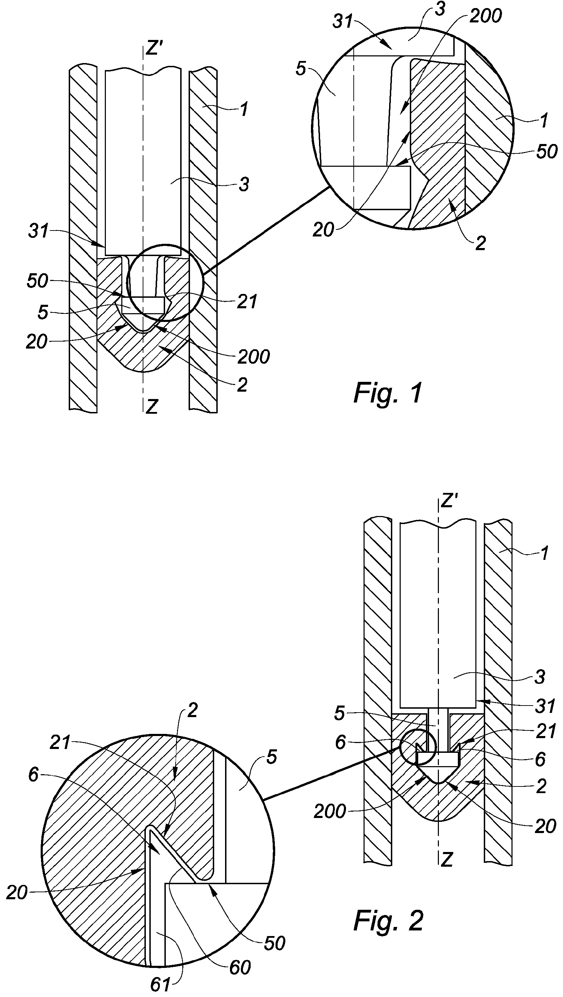

Une telle seringue de l'état de la technique n'est pas entièrement satisfaisante dans la mesure où la tête d'encliquetage est susceptible de s'extraire de la cavité du butoir lorsque le médecin souhaite vérifier que l'aiguille ne se trouve pas dans une veine du patient, dans le cadre d'une application de type injection par voie intradermique. Pour ce faire, le médecin procède à une aspiration en tirant sur le poussoir, suivant le sens opposé au sens d'injection du produit, sur une distance classiquement comprise entre 5 mm et 10 mm, et ce afin de vérifier l'absence d'un reflux sanguin. Ce geste de vérification du médecin est une précaution importante puisque toute injection sous le plan dermique augmente considérablement le risque d'injection intravasculaire. Ce geste de vérification du médecin conduit à exercer une force -dite d'arrachement- importante (par exemple de l'ordre de 6 à 8 N) sur la liaison entre la surface d'appui plane de la tête d'encliquetage et la paroi interne du butoir, tout particulièrement lorsque le produit à injecter est visqueux (effet de succion). Une telle intensité de force, s'exerçant sur la liaison entre la surface d'appui plane de la tête d'encliquetage et la paroi interne du butoir, peut provoquer une déformation de la paroi interne du butoir si importante que la tête d'encliquetage peut alors s'extraire de la cavité du butoir, comme illustré à la figure 1.

L'homme du métier recherche donc une seringue permettant de résister à une force d'arrachement élevée, par exemple supérieure ou égale à 9 N, afin de sécuriser le geste de vérification du médecin.

WO 2008/064283 A2 décrit un piston, et un distributeur manuel comprenant un piston, à utiliser pour distribuer des matériaux tels que des adhésifs dentaires ou des produits de restauration à la main. EP 0 354 368 A2 décrit des ampoules jetables pour seringues hypodermiques. WO 03/047668 A1 décrit un dispositif d'administration de médicaments ou d'autres substances fluides dans le corps d'un mammifère. WO 2011/023738 A1 décrit un ensemble de seringue et, en particulier, un ensemble de seringue jetable préremplie destiné à l'administration d'un produit médicamenteux liquide à un patient, de préférence par injection. US 2011/034882 A1 décrit un ensemble de butoir destiné à être utilisé avec une seringue et, plus particulièrement, un ensemble de butoir ayant un faible espace mort et pratiquement aucun reflux, destiné à être utilisé avec une seringue préremplie, telle que celles utilisées dans les applications de rinçage.

### Exposé de l'invention

L'invention vise à remédier en tout ou partie aux inconvénients précités. A cet effet, l'invention a pour objet une seringue, telle que décrite dans la revendication 1 annexée.

### Définitions

- Par « paroi interne », on entend la surface interne du butoir délimitant une cavité, le butoir étant creux.
- Par « élastiquement déformable », on entend que la paroi interne du butoir peut se déformer de manière réversible sous l'action de contraintes mécaniques (inférieures à la limite élastique), c'est-à-dire que la paroi interne du butoir peut revenir à sa forme initiale lorsque les contraintes mécaniques cessent.
- Par « encliquetage », on entend un emboîtage élastique entre la tête d'encliquetage et la paroi interne du butoir, à l'intérieur de la cavité du butoir.
- Par « saillant », on entend que l'élément dépasse de la surface d'appui plane de manière à s'étendre dans un demi-espace défini par la surface d'appui plane, le demi-espace s'étendant vers la seconde extrémité de la tige.

Ainsi, une telle seringue selon l'invention permet de résister à une force d'arrachement plus importante que dans l'état de la technique, et ce grâce à l'élément saillant ou aux éléments saillants qui vont s'ancrer à la paroi interne du butoir (en la déformant localement) lorsque le piston coulisse suivant le sens opposé au sens d'injection du produit, en particulier lors de l'exécution du geste de vérification par le médecin, comme illustré à la figure 2.

La seringue selon l'invention peut comporter une ou plusieurs des caractéristiques suivantes.

Selon une caractéristique de l'invention, le ou chaque élément saillant comporte deux surfaces planes formant un angle dièdre aigu, de préférence compris entre 10° et 80°, plus préférentiellement compris entre 30° et 60°, encore plus préférentiellement compris entre 40° et 50°.

### Définition

Par « angle dièdre aigu », on entend l'angle entre deux plans définis par les deux surfaces planes, compris entre 0° et 90°.

Ainsi, un avantage procuré par de telles surfaces planes est de former une partie pointue permettant à l'élément saillant de pénétrer dans la paroi interne du butoir lorsque le piston coulisse suivant le sens opposé au sens d'injection du produit, de manière à s'y ancrer en la déformant localement. En d'autres termes, de telles surfaces planes forment une pointe d'ancrage.

Selon une caractéristique de l'invention, les deux surfaces planes du ou de chaque élément saillant sont réunies de manière à former une arête.

### Définition

Par « former une arête », on entend la formation d'une ligne d'intersection entre les deux surfaces planes. En d'autres termes, les deux surfaces planes du ou de chaque élément saillant sont réunies de manière à former un bord vif.

Ainsi, un avantage procuré est de faciliter la pénétration de l'élément saillant dans la paroi interne du butoir.

Selon une caractéristique de l'invention, le ou chaque élément saillant comporte une surface conique ou tronconique présentant un angle de cône aigu, de préférence compris entre 10° et 80°, plus préférentiellement compris entre 30° et 60°, encore plus préférentiellement compris entre 40° et 50°.

### Définitions

- Par « surface conique », on entend la surface engendrée par un segment (génératrice) passant par un point fixe (sommet) et par un point variable décrivant une courbe (directrice). A titre d'exemples non limitatifs, la courbe directrice peut être un cercle, une ellipse, un polygone.
- Par « surface tronconique », on entend une partie de la surface conique comprise entre la base et une section plane parallèle à la base.
- Par « angle de cône aigu », on entend le demi-angle au sommet du cône, compris entre 0° et 90°.

Ainsi, un avantage procuré par une telle surface conique ou tronconique est de former une partie pointue permettant à l'élément saillant de pénétrer dans la paroi interne du butoir lorsque le piston coulisse suivant le sens opposé au sens d'injection du produit, de manière à s'y ancrer en la déformant localement. En d'autres termes, une telle surface conique ou tronconique forme une pointe d'ancrage.

Selon une caractéristique de l'invention, le ou chaque élément saillant forme une dent présentant un angle de pointe aigu, de préférence compris entre 10° et 80°, plus préférentiellement compris entre 30° et 60°, encore plus préférentiellement compris entre 40° et 50°.

### Définitions

- Par « dent », on entend deux flancs réunis de manière à former une surface de crête.
- Par « angle de pointe aigu », on entend l'angle dièdre formé entre les deux flancs de la dent, l'angle dièdre étant compris entre 0° et 90°.

Ainsi, un avantage procuré par une telle dent est de former une partie pointue permettant à l'élément saillant de pénétrer dans la paroi interne du butoir lorsque le piston coulisse suivant le sens opposé au sens d'injection du produit, de manière à s'y ancrer en la déformant localement. En d'autres termes, une telle dent forme une pointe d'ancrage.

Selon l'invention, la surface d'appui plane est située à une distance, notée D, de la seconde extrémité de la tige, et le ou chaque élément saillant présente une hauteur, notée H, suivant la normale à la surface d'appui plane vérifiant :
0,05 D ≤ H ≤ 0,2 D, de préférence 0,06 D ≤ H ≤ 0,18 D.

Ainsi, un avantage procuré est d'obtenir une profondeur de pénétration de l'élément saillant dans la paroi interne du butoir permettant un ancrage suffisant pour résister à une force d'arrachement élevée (par exemple supérieure ou égale à 9 N), tout en conservant une force d'encliquetage pas trop élevée (par exemple inférieure à 8 N) afin d'emboîter la tête d'encliquetage avec la paroi interne du butoir à l'intérieur de la cavité du butoir.

Selon une caractéristique de l'invention, la seringue comporte un ensemble d'éléments saillants s'étendant en saillie de la surface d'appui plane, et agencés pour pénétrer dans la paroi interne du butoir lorsque le piston coulisse suivant le sens opposé au sens d'injection du produit.

Ainsi, un avantage procuré est d'augmenter la résistance à la force d'arrachement en augmentant le nombre d'éléments saillants.

Selon une caractéristique de l'invention, les éléments saillants de l'ensemble forment des motifs espacés de manière périodique sur la surface d'appui plane.

### Définition

Par « espacés de manière périodique », on entend que les motifs se répètent (de préférence à l'identique) selon un pas donné, c'est-à-dire selon une période spatiale donnée.

Ainsi, un avantage procuré est d'augmenter l'adhérence des éléments saillants à la paroi interne du butoir (effet de moletage), et par là-même d'augmenter la résistance à la force d'arrachement.

Selon une caractéristique de l'invention, la surface d'appui plane est circulaire, et l'ensemble d'éléments saillants est réparti uniformément autour de la surface d'appui plane.

### Définition

Par « réparti uniformément », on entend que les éléments saillants de l'ensemble sont agencés selon une distance radiale invariable par rapport au centre de la surface plane circulaire, et sont distribués autour de la surface d'appui plane selon une loi uniforme de manière à former un angle de 2π/N radians, où N est le nombre d'éléments saillants de l'ensemble.

Ainsi, un avantage procuré par une telle géométrie est d'augmenter la résistance à la force d'arrachement.

Selon une caractéristique de l'invention, les éléments saillants de l'ensemble présentent une symétrie axiale par rapport à l'axe longitudinal.

Ainsi, un avantage procuré par une telle géométrie est d'augmenter la résistance à la force d'arrachement.

Selon une caractéristique de l'invention, la surface d'appui plane présente un rayon, noté R, et le ou chaque élément saillant présente une extrémité latérale située à une distance radiale, notée r, vérifiant :
0,8 R ≤ r ≤ 1,20 R, de préférence R ≤ r ≤ 1,16 R.

### Définition

Par « distance radiale », on entend la distance de l'extrémité latérale par rapport au centre de la surface d'appui plane.

Ainsi, un avantage procuré est de limiter la force d'encliquetage nécessaire pour emboîter la tête d'encliquetage avec la paroi interne du butoir à l'intérieur de la cavité du butoir, tout en réduisant les risques de briser les extrémités latérales des éléments saillants lors de l'encliquetage.

Selon une caractéristique de l'invention, le ou chaque élément saillant et la tête d'encliquetage sont monobloc.

Ainsi, un avantage procuré est de faciliter leur fabrication, qui peut être exécutée par moulage.

Selon une caractéristique de l'invention, la tête d'encliquetage et le ou les éléments saillants sont réalisés dans un matériau plastique, de préférence un thermoplastique, plus préférentiellement le polycarbonate.

Selon une caractéristique de l'invention, le butoir est réalisé dans un matériau élastomère, de préférence un caoutchouc butyle chloré ou bromé.

Selon une caractéristique de l'invention, la paroi interne du butoir comporte au moins un épaulement contre lequel vient en appui la surface d'appui plane de la tête d'encliquetage lorsque le piston coulisse suivant le sens opposé au sens d'injection du produit ; et le ou les éléments saillants sont agencés pour pénétrer dans l'épaulement lorsque le piston coulisse suivant le sens opposé au sens d'injection du produit.

Ainsi, un avantage procuré par l'épaulement est de former un appui pour la tête d'encliquetage lorsque le piston coulisse suivant le sens opposé au sens d'injection du produit.

### Brève description des dessins

D'autres caractéristiques et avantages apparaîtront dans l'exposé détaillé de différents modes de réalisation de l'invention, l'exposé étant assorti d'exemples et de références aux dessins joints.
Figure 1 est une vue schématique partielle, en coupe, d'une seringue selon l'état de la technique, où l'encart illustre une déformation importante de la paroi interne du butoir, la tête d'encliquetage pouvant alors s'extraire de la cavité du butoir lorsque la force d'arrachement est élevée.
Figure 2 est une vue schématique partielle, en coupe, d'une seringue selon l'invention, où l'encart illustre un élément saillant qui s'ancre à la paroi interne du butoir (en la déformant localement) lorsque le piston coulisse suivant le sens opposé au sens d'injection du produit.

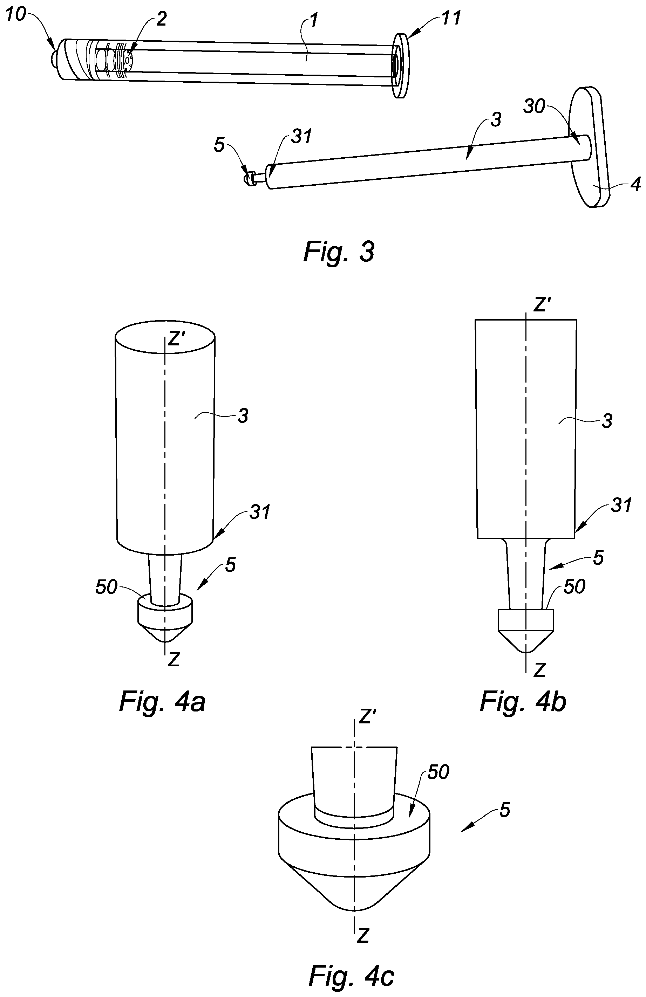

Figure 3 est une vue schématique, en perspective, d'une seringue selon l'invention, illustrant séparément le corps de seringue muni d'un butoir et le piston muni d'une tête d'encliquetage (les éléments saillants ne sont pas clairement visibles de par leurs dimensions).
Figures 4a à 4c sont des vues schématiques (respectivement en perspective, en coupe et à l'échelle agrandie) d'une tête d'encliquetage équipant une seringue de l'état de la technique.

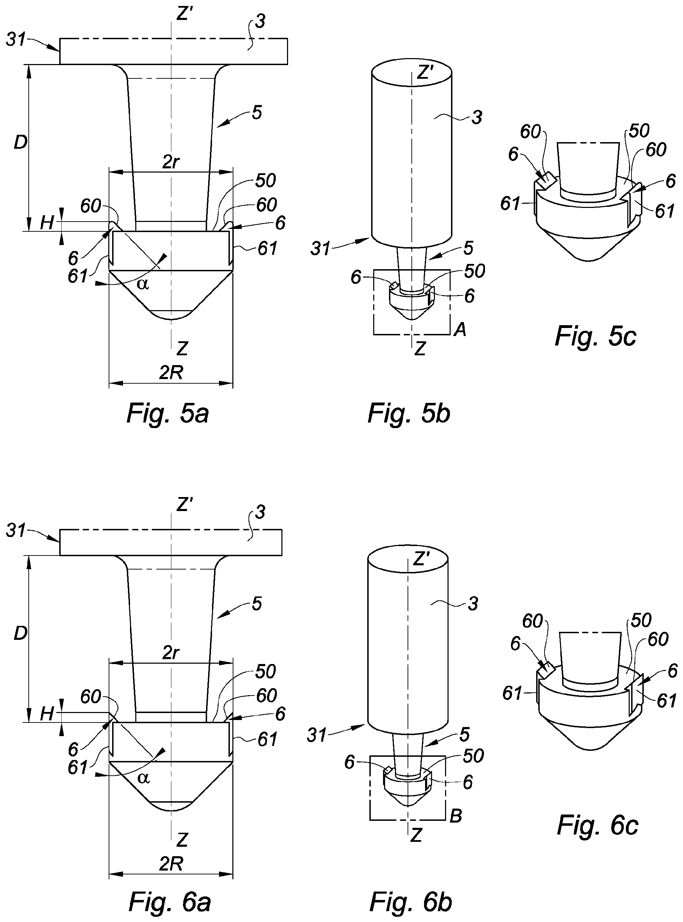

Figures 5a à 5c sont des vues schématiques (respectivement en coupe à l'échelle agrandie, en perspective et en perspective à l'échelle agrandie) d'une tête d'encliquetage équipant une seringue selon l'invention, illustrant un premier mode de réalisation des éléments saillants. La figure 5c est une vue en perspective à l'échelle agrandie de la zone A de la figure 5b.
Figures 6a à 6c sont des vues schématiques (respectivement en coupe à l'échelle agrandie, en perspective et en perspective à l'échelle agrandie) d'une tête d'encliquetage équipant une seringue selon l'invention, illustrant un deuxième mode de réalisation des éléments saillants. La figure 6c est une vue en perspective à l'échelle agrandie de la zone B de la figure 6b.

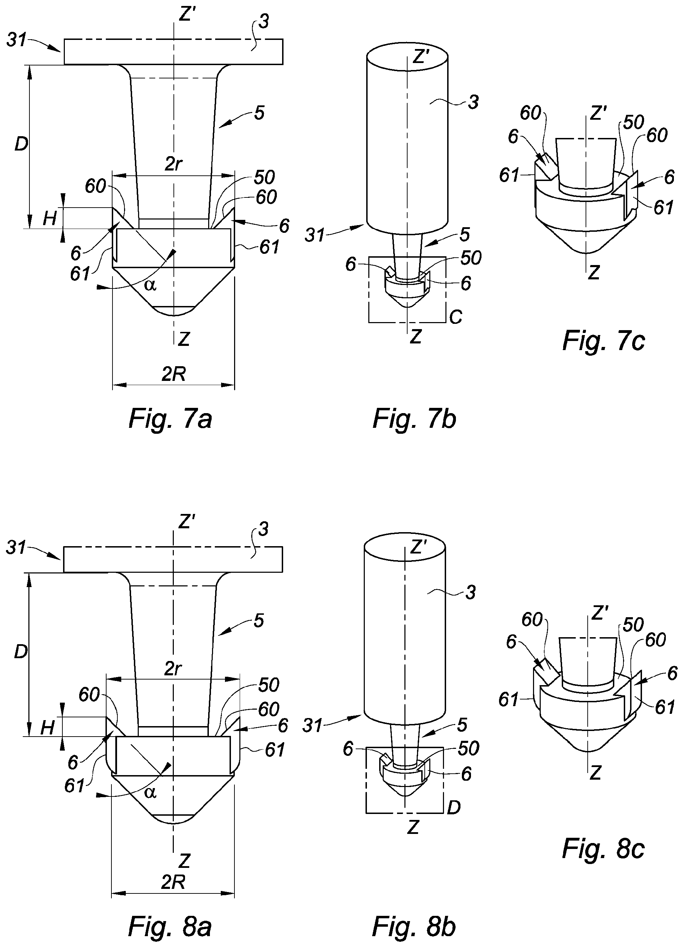

Figures 7a à 7c sont des vues schématiques (respectivement en coupe à l'échelle agrandie, en perspective et en perspective à l'échelle agrandie) d'une tête d'encliquetage équipant une seringue selon l'invention, illustrant un troisième mode de réalisation des éléments saillants. La figure 7c est une vue en perspective à l'échelle agrandie de la zone C de la figure 7b.
Figures 8a à 8c sont des vues schématiques (respectivement en coupe à l'échelle agrandie, en perspective et en perspective à l'échelle agrandie) d'une tête d'encliquetage équipant une seringue selon l'invention, illustrant un quatrième mode de réalisation des éléments saillants. La figure 8c est une vue en perspective à l'échelle agrandie de la zone D de la figure 8b.

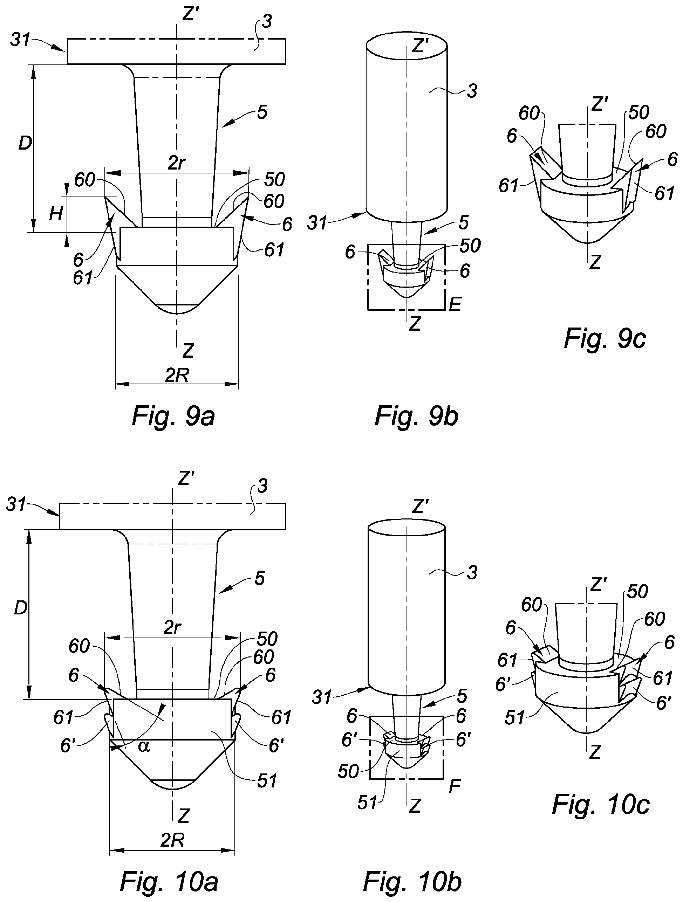

Figures 9a à 9c sont des vues schématiques (respectivement en coupe à l'échelle agrandie, en perspective et en perspective à l'échelle agrandie) d'une tête d'encliquetage équipant une seringue selon l'invention, illustrant un cinquième mode de réalisation des éléments saillants. La figure 9c est une vue en perspective à l'échelle agrandie de la zone E de la figure 9b.
Figures 10a à 10c sont des vues schématiques (respectivement en coupe à l'échelle agrandie, en perspective et en perspective à l'échelle agrandie) d'une tête d'encliquetage équipant une seringue selon l'invention, illustrant un sixième mode de réalisation des éléments saillants. La figure 10c est une vue en perspective à l'échelle agrandie de la zone F de la figure 10b.

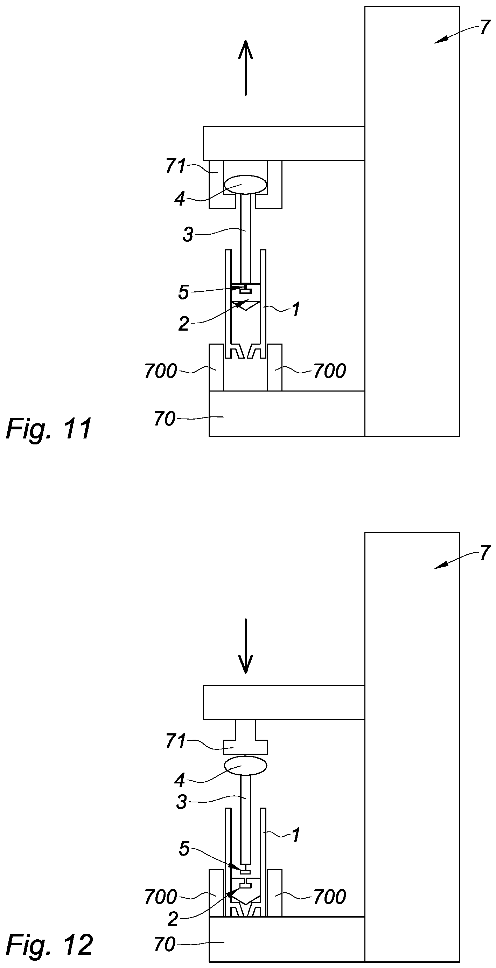

Figure 11 est un schéma synoptique (en coupe) d'un banc de test permettant de mesurer la force d'arrachement permettant d'extraire la tête d'encliquetage hors de la paroi interne du butoir.
Figure 12 est un schéma synoptique (en coupe) d'un banc de test permettant de mesurer la force d'encliquetage nécessaire pour un emboîtage de la tête d'encliquetage à l'intérieur de la cavité du butoir.

Il est à noter que les dessins décrits ci-avant sont schématiques, et ne sont pas nécessairement à l'échelle par souci de lisibilité et pour en simplifier leur compréhension. Les coupes sont effectuées suivant l'axe longitudinal de la tige.

### Exposé détaillé des modes de réalisation

Les éléments identiques ou assurant la même fonction porteront les mêmes références pour les différents modes de réalisation, par souci de simplification.

Un objet de l'invention est une seringue, comportant :
- un corps 1 de seringue, destiné à recevoir un produit à injecter, et comprenant un butoir 2 ; le butoir 2 présentant une paroi interne 20, élastiquement déformable, et délimitant une cavité 200 ;
- un piston, monté coulissant à l'intérieur du corps de seringue suivant un sens d'injection du produit et suivant un sens opposé, et comprenant une tige 3 présentant une première extrémité 30 et une seconde extrémité 31 opposée ;
- un poussoir 4, agencé à la première extrémité 30 de la tige 3 ;
- une tête 5 d'encliquetage, agencée à la seconde extrémité 31 de la tige 3, et encliquetée à l'intérieur de la cavité 200 du butoir 2 ; la tête 5 d'encliquetage présentant une surface d'appui 50 plane venant en appui contre la paroi interne 20 du butoir 2 lorsque le piston coulisse suivant le sens opposé au sens d'injection du produit ;
la tête 5 d'encliquetage comporte au moins un élément saillant 6, s'étendant en saillie de la surface d'appui 50 plane, et agencé pour pénétrer dans la paroi interne 20 du butoir 2 lorsque le piston coulisse suivant le sens opposé au sens d'injection du produit.

Différentes formes et différents agencements du ou des éléments saillants sont illustrés aux figures 5a à 5c, 6a à 6c, 7a à 7c, 8a à 8c, 9a à 9c, 10a à 10c.

### Corps de seringue

Le corps 1 de seringue présente :
- une première extrémité 10, destinée à être équipée d'un porte-aiguille ;
- une seconde extrémité 11, opposée.

Les première et seconde extrémités 10, 11 du corps 1 de seringue sont ouvertes. La première extrémité 10 du corps 1 de seringue est ouverte pour recevoir un porte-aiguille. En l'absence de porte-aiguille, la première extrémité 10 du corps 1 de seringue peut être obstruée par un capuchon. La seconde extrémité 11 du corps 1 de seringue est ouverte pour recevoir le piston. La seringue comporte avantageusement des ailettes de préhension, agencées à la seconde extrémité 11 du corps 1 de seringue.

Le corps 1 de seringue est de préférence cylindrique. Le corps 1 de seringue est de préférence réalisé dans un matériau plastique, plus préférentiellement un matériau thermoplastique. A titre d'exemple non limitatif, le corps 1 de seringue peut être réalisé en polycarbonate.

A titre d'exemple non limitatif, le produit à injecter peut être un gel viscoélastique à base d'acide hyaluronique.

### Butoir

La paroi interne 20 du butoir 2 comporte avantageusement au moins un épaulement 21 contre lequel vient en appui la surface d'appui 50 plane de la tête 5 d'encliquetage lorsque le piston coulisse suivant le sens opposé au sens d'injection du produit.

Lorsque la tête 5 d'encliquetage est encliquetée à l'intérieur de la cavité 200 du butoir 2, le butoir 2 forme une tête de piston. A titre d'exemple non limitatif, lorsque le butoir 2 est en position de fin de course, la tige 3 peut être en saillie du corps 1 de seringue sur une distance comprise entre 20 mm et 30 mm.

Le butoir 2 est avantageusement réalisé dans un matériau élastomère, de préférence un caoutchouc butyle chloré ou bromé. Le butoir 2 est réalisé dans un matériau mou, présentant avantageusement une dureté comprise entre 45 Shore A et 60 Shore A.

### Tige de piston

La tige 3 est de préférence réalisée dans un matériau plastique, plus préférentiellement un matériau thermoplastique. A titre d'exemple non limitatif, la tige 3 peut être réalisée en polycarbonate. La tige 3 s'étend suivant un axe longitudinal Z'-Z.

### Poussoir

Le poussoir 4 et la tige 3 peuvent être monobloc. Le poussoir 4 présente avantageusement une masse inférieure ou égale à 10 grammes afin de ne pas alourdir inutilement la seringue.

### Tête d'encliquetage

La surface d'appui 50 plane est située à une distance, notée D, de la seconde extrémité 31 de la tige 3. A titre d'exemple non limitatif, la distance D peut être comprise entre 3 mm et 4 mm, de préférence comprise entre 3,2 mm et 3,6 mm. La surface d'appui 50 plane peut être circulaire, et présenter un rayon, noté R. A titre d'exemple non limitatif, le rayon R peut être compris entre 1 mm et 1,5 mm.

La tête 5 d'encliquetage s'étend suivant l'axe longitudinal Z'-Z.

Le ou chaque élément saillant 6 et la tête 5 d'encliquetage sont avantageusement monobloc, de préférence obtenus par moulage. La tête 5 d'encliquetage et le ou les éléments saillants 6 sont avantageusement réalisés dans un matériau plastique, de préférence un thermoplastique, plus préférentiellement le polycarbonate. La tête 5 d'encliquetage et le ou les éléments saillants 6 sont réalisés dans un matériau dur, présentant avantageusement une dureté comprise entre 60 Shore D et 90 Shore D.

### Forme du ou des éléments saillants

Selon un mode de réalisation, le ou chaque élément saillant 6 comporte deux surfaces planes 60, 61 formant un angle dièdre α aigu, de préférence compris entre 10° et 80°, plus préférentiellement compris entre 30° et 60°, encore plus préférentiellement compris entre 40° et 50°. Comme illustré aux figures 6a à 6c, 7a à 7c, 8a à 8c, 9a à 9c, les deux surfaces planes 60, 61 du ou de chaque élément saillant 6 peuvent être réunies de manière à former une arête, c'est-à-dire un bord vif. Selon une alternative illustrée aux figures 5a à 5c et 10a à 10c, les deux surfaces planes 60, 61 du ou de chaque élément saillant 6 peuvent être réunies de manière à former un bord arrondi. La ligne d'intersection (réelle ou virtuelle) de l'angle dièdre α est orientée du côté de la tige 3.

Selon un mode de réalisation non illustré, le ou chaque élément saillant 6 comporte une surface conique ou tronconique présentant un angle de cône aigu, de préférence compris entre 10° et 80°, plus préférentiellement compris entre 30° et 60°, encore plus préférentiellement compris entre 40° et 50°. Le sommet (réel ou virtuel) du cône de la surface conique ou tronconique est orienté du côté de la tige 3.

Selon un mode de réalisation non illustré, le ou chaque élément saillant 6 forme une dent présentant un angle de pointe aigu, de préférence compris entre 10° et 80°, plus préférentiellement compris entre 30° et 60°, encore plus préférentiellement compris entre 40° et 50°. La surface de crête de la dent est orientée du côté de la tige 3.

### Agencement du ou des éléments saillants

Le ou chaque élément saillant 6 présente une hauteur, notée H, suivant la normale à la surface d'appui 50 plane vérifiant :
0,05 D ≤ H ≤ 0,2 D, de préférence 0,06 D ≤ H ≤ 0,18 D.

Lorsque la surface d'appui 50 plane de la tête 5 d'encliquetage est circulaire, le ou chaque élément saillant 6 présente une extrémité latérale située à une distance radiale, notée r, vérifiant :
0,8 R ≤ r ≤ 1,20 R, de préférence R ≤ r ≤ 1,16 R.

La seringue peut comporter un ensemble d'éléments saillants 6 s'étendant en saillie de la surface d'appui 50 plane, et agencés pour pénétrer dans la paroi interne 20 du butoir 2 lorsque le piston coulisse suivant le sens opposé au sens d'injection du produit.

Selon un mode de réalisation non illustré, les éléments saillants 6 de l'ensemble peuvent former des motifs espacés de manière périodique sur la surface d'appui 50 plane. A titre d'exemple non limitatif, les motifs peuvent être agencés sur la surface d'appui 50 plane de manière à délimiter des stries radiales et former un moletage.

Lorsque la surface d'appui 50 plane est circulaire, l'ensemble d'éléments saillants 6 est avantageusement réparti uniformément autour de la surface d'appui 50 plane. Il est possible d'avoir un nombre pair ou un nombre impair d'éléments saillants 6 répartis uniformément autour de la surface d'appui 50 plane.

Les éléments saillants 6 de l'ensemble présentent avantageusement une symétrie axiale par rapport à l'axe longitudinal Z'-Z de la tête 5 d'encliquetage.

Dans le cas où la paroi interne 20 du butoir 2 comporte au moins un épaulement 21 contre lequel vient en appui la surface d'appui 50 plane de la tête 5 d'encliquetage lorsque le piston coulisse suivant le sens opposé au sens d'injection du produit, le ou les éléments saillants 6 sont alors avantageusement agencés pour pénétrer dans l'épaulement 21 lorsque le piston coulisse suivant le sens opposé au sens d'injection du produit.

Comme illustré aux figures 10a à 10c, la tête 5 d'encliquetage peut comporter au moins un élément saillant 6' additionnel, s'étendant en saillie d'une bordure latérale 51 de la tête 5 d'encliquetage, et agencé pour pénétrer dans la paroi interne 20 du butoir 2 lorsque le piston coulisse suivant le sens opposé au sens d'injection du produit.

### Bancs de test

Le banc de test 7 schématiquement illustré à la figure 11 permet de mesurer la force d'arrachement pour extraire la tête 5 d'encliquetage hors de la paroi interne 20 du butoir 2. Le banc de test 7 comporte une platine 70 comprenant des parois 700, formant une mâchoire, et agencées pour maintenir en position la seringue en l'enserrant. Le butoir 2 est fixé au corps 1 de seringue (par exemple à la première extrémité 10 du corps 1 de seringue), de préférence à l'aide d'une colle forte. Le banc de test 7 comporte un actionneur 71 agencé pour tirer le poussoir 4 (mouvement de traction) jusqu'à l'arrachement de la tête 5 d'encliquetage hors du butoir 2. L'actionneur 71 est configuré pour se déplacer selon une vitesse représentative de la vitesse d'exécution du geste de vérification du médecin, sur une distance prédéterminée. L'effort généré par l'actionneur 71 est mesuré par un capteur durant le mouvement de traction. La valeur maximale de l'effort généré par l'actionneur 71 est retenue comme la valeur de force d'arrachement.

Le banc de test 7 schématiquement illustré à la figure 12 permet de mesurer la force d'encliquetage nécessaire pour un emboîtage de la tête 5 d'encliquetage à l'intérieur de la cavité 200 du butoir 2. Le banc de test 7 comporte une platine 70 comprenant des parois 700, formant une mâchoire, et agencées pour maintenir en position la seringue en l'enserrant. Le butoir 2 est introduit au fond du corps 1 de seringue (à la première extrémité 10 du corps 1 de seringue). Le banc de test 7 comporte un actionneur 71 agencé pour pousser sur le poussoir 4 (mouvement de compression) jusqu'à l'encliquetage de la tête 5 d'encliquetage à l'intérieur de la cavité 200 du butoir 2. L'effort généré par l'actionneur 71 est mesuré par un capteur durant le mouvement de compression. L'encliquetage de la tête 5 d'encliquetage à l'intérieur de la cavité 200 du butoir 2 conduit à un pic d'effort généré par l'actionneur 71. La valeur maximale du pic d'effort généré est mesurée, et retenue comme la valeur de force d'encliquetage.

Pour une seringue selon l'invention, la force d'arrachement mesurée peut être comprise entre 11,8 N et 12,5 N, avec une force d'encliquetage mesurée comprise entre 6,8 N et 8,6 N.

L'invention ne se limite pas aux modes de réalisation exposés. L'homme du métier est mis à même de considérer leurs combinaisons techniquement opérantes, et de leur substituer des équivalents.

## Revendications

1. Seringue, comportant :
- un corps (1) de seringue, destiné à recevoir un produit à injecter, et comprenant un butoir (2) ; le butoir (2) présentant une paroi interne (20), élastiquement déformable, et délimitant une cavité (200) ;
- un piston, monté coulissant à l'intérieur du corps (1) de seringue suivant un sens d'injection du produit et suivant un sens opposé, et comprenant une tige (3) présentant une première extrémité (30) et une seconde extrémité (31) opposée ;
- un poussoir (4), agencé à la première extrémité (30) de la tige (3) ;
- une tête (5) d'encliquetage, s'étendant suivant un axe longitudinal (Z'-Z), agencée à la seconde extrémité (31) de la tige (3), et encliquetée à l'intérieur de la cavité (200) du butoir (2) par emboîtage élastique avec la paroi interne (20) du butoir (2) ; la tête (5) d'encliquetage présentant une surface d'appui (50) plane ; la surface d'appui (50) plane est située à une distance, notée D, de la seconde extrémité (31) de la tige (3) ; la surface d'appui (50) plane définissant un demi-espace s'étendant vers la seconde extrémité (31) de la tige (3) ;
la tête (5) d'encliquetage comporte au moins un élément saillant (6), s'étendant en saillie de la surface d'appui (50) plane, et agencé pour pénétrer dans la paroi interne (20) du butoir (2) lorsque le piston coulisse suivant le sens opposé au sens d'injection du produit de manière à s'ancrer à la paroi interne (20) du butoir (2) en la déformant localement ;
**caractérisée en ce que** :
- la surface d'appui (50) plane vient en appui contre la paroi interne (20) du butoir (2) lorsque le piston coulisse suivant le sens opposé au sens d'injection du produit ;
- le ou chaque élément saillant (6) s'étend dans le demi-espace, et **en ce que** le ou chaque élément saillant (6) présente une hauteur, notée H, suivant la normale à la surface d'appui (50) plane vérifiant 0,05 D ≤ H ≤ 0,2 D.

2. Seringue selon la revendication 1, dans laquelle le ou chaque élément saillant (6) comporte deux surfaces planes (60, 61) formant un angle dièdre (α) aigu, de préférence compris entre 10° et 80°, plus préférentiellement compris entre 30° et 60°, encore plus préférentiellement compris entre 40° et 50°.

3. Seringue selon la revendication 2, dans laquelle les deux surfaces planes (60, 61) du ou de chaque élément saillant (6) sont réunies de manière à former une arête.

4. Seringue selon la revendication 1, dans laquelle le ou chaque élément saillant (6) comporte une surface conique ou tronconique présentant un angle de cône aigu, de préférence compris entre 10° et 80°, plus préférentiellement compris entre 30° et 60°, encore plus préférentiellement compris entre 40° et 50°.

5. Seringue selon la revendication 1, dans laquelle le ou chaque élément saillant (6) forme une dent présentant un angle de pointe aigu, de préférence compris entre 10° et 80°, plus préférentiellement compris entre 30° et 60°, encore plus préférentiellement compris entre 40° et 50°.

6. Seringue selon l'une des revendications 1 à 5, comportant un ensemble d'éléments saillants (6) s'étendant en saillie de la surface d'appui (50) plane, et agencés pour pénétrer dans la paroi interne (20) du butoir (2) lorsque le piston coulisse suivant le sens opposé au sens d'injection du produit.

7. Seringue selon la revendication 6, dans laquelle les éléments saillants (6) de l'ensemble forment des motifs espacés de manière périodique sur la surface d'appui (50) plane.

8. Seringue selon la revendication 6 ou 7, dans laquelle la surface d'appui (50) plane est circulaire, et l'ensemble d'éléments saillants (6) est réparti uniformément autour de la surface d'appui (50) plane.

9. Seringue selon l'une des revendications 6 à 8, dans laquelle les éléments saillants (6) de l'ensemble présentent une symétrie axiale par rapport à l'axe longitudinal (Z'-Z).

10. Seringue selon l'une des revendications 1 à 9, dans laquelle la surface d'appui (50) plane présente un rayon, noté R, et le ou chaque élément saillant (6) présente une extrémité latérale située à une distance radiale, notée r, vérifiant:
0,8 R ≤ r ≤ 1,20 R, de préférence R ≤ r ≤ 1,16 R.

11. Seringue selon l'une des revendications 1 à 10, dans laquelle le ou chaque élément saillant (6) et la tête (5) d'encliquetage sont monobloc.

12. Seringue selon l'une des revendications 1 à 11, dans laquelle la tête (5) d'encliquetage et le ou les éléments saillants (6) sont réalisés dans un matériau plastique, de préférence un thermoplastique, plus préférentiellement le polycarbonate.

13. Seringue selon l'une des revendications 1 à 12, dans laquelle le butoir (2) est réalisé dans un matériau élastomère, de préférence un caoutchouc butyle chloré ou bromé.

14. Seringue selon l'une des revendications 1 à 13, dans laquelle la paroi interne (20) du butoir (2) comporte au moins un épaulement (21) contre lequel vient en appui la surface d'appui (50) plane de la tête (5) d'encliquetage lorsque le piston coulisse suivant le sens opposé au sens d'injection du produit ; et le ou les éléments saillants (6) sont agencés pour pénétrer dans l'épaulement (21) lorsque le piston coulisse suivant le sens opposé au sens d'injection du produit.

## Patentansprüche

1. Spritze, umfassend:
- einen Spritzenkörper (1), der dazu bestimmt ist, ein zu injizierendes Produkt aufzunehmen und einen Anschlag (2) umfasst; wobei der Anschlag (2) eine Innenwand (20) aufweist, die elastisch verformbar ist, und einen Hohlraum (200) begrenzt;
- einen Kolben, der im Inneren des Spritzenkörpers (1) entlang einer Injektionsrichtung des Produkts und entlang einer entgegengesetzten Richtung verschiebbar gelagert ist und eine Stange (3) umfasst, die ein erstes Ende (30) und ein entgegengesetztes zweites Ende (31) aufweist;
- ein Druckstück (4), das an dem ersten Ende (30) der Stange (3) angeordnet ist;
- einen Rastkopf (5), der sich entlang einer Längsachse (Z'-Z) erstreckt, an dem zweiten Ende (31) der Stange (3) angeordnet ist und im Inneren des Hohlraums (200) des Anschlags (2) durch eine Schnappverbindung mit der Innenwand (20) des Anschlags (2) eingerastet ist; wobei der Rastkopf (5) eine plane Anlagefläche (50) aufweist; wobei die plane Anlagefläche (50) in einem mit D bezeichneten Abstand von dem zweiten Ende (31) der Stange (3) gelegen ist; wobei die plane Anlagefläche (50) einen Halbraum definiert, der sich zu dem zweiten Ende (31) der Stange (3) hin erstreckt;
der Rastkopf (5) umfasst mindestens ein vorspringendes Element (6), das sich von der planen Anlagefläche (50) vorspringend erstreckt und so gestaltet ist, dass es in die Innenwand (20) des Anschlags (2) eindringt, wenn der Kolben entlang der zur Injektionsrichtung des Produkts entgegengesetzten Richtung gleitet, so dass es sich an der Innenwand (20) des Anschlags (2) verankert, indem es sie lokal verformt;
**dadurch gekennzeichnet, dass**:
- die plane Anlagefläche (50) an der Innenwand (20) des Anschlags (2) zur Anlage gelangt, wenn der Kolben entlang der zur Injektionsrichtung des Produkts entgegengesetzten Richtung gleitet;
- das oder jedes vorspringende Element (6) sich in dem Halbraum erstreckt und dass das oder jedes vorspringende Element (6) eine mit H bezeichnete Höhe entlang der Normalen zu der planen Anlagefläche (50) aufweist, für die 0,05 D ≤ H ≤ 0,2 D gilt.

2. Spritze nach Anspruch 1, wobei das oder jedes vorspringende Element (6) zwei plane Flächen (60, 61) umfasst, die einen spitzen Flächenwinkel (α) von bevorzugt zwischen 10° und 80°, noch bevorzugter zwischen 30° und 60°, noch stärker bevorzugt zwischen 40° und 50°, bilden.

3. Spritze nach Anspruch 2, wobei die beiden planen Flächen (60, 61) des oder jedes vorspringenden Elements (6) so verbunden sind, dass sie eine Kante bilden.

4. Spritze nach Anspruch 1, wobei das oder jedes vorspringende Element (6) eine konische oder kegelstumpfförmige Fläche umfasst, die einen spitzen Kegelwinkel von bevorzugt zwischen 10° und 80°, noch bevorzugter zwischen 30° und 60°, noch stärker bevorzugt zwischen 40° und 50°, aufweist.

5. Spritze nach Anspruch 1, wobei das oder jedes vorspringende Element (6) eine Zacke bildet, die einen spitzen Spitzenwinkel von bevorzugt zwischen 10° und 80°, noch bevorzugter zwischen 30° und 60°, noch stärker bevorzugt zwischen 40° und 50°, aufweist.

6. Spritze nach einem der Ansprüche 1 bis 5, umfassend eine Anordnung vorspringender Elemente (6), die sich von der planen Anlagefläche (50) vorspringend erstecken und so gestaltet sind, dass sie in die Innenwand (20) des Kolbens (2) eindringen, wenn der Kolben entlang der zur Injektionsrichtung des Produkts entgegengesetzten Richtung gleitet.

7. Spritze nach Anspruch 6, wobei die vorspringenden Elemente (6) der Anordnung Muster bilden, die auf der planen Anlagefläche (50) periodisch beabstandet sind.

8. Spritze nach Anspruch 6 oder 7, wobei die plane Anlagefläche (50) kreisförmig ist und die Anordnung vorspringender Elemente (6) gleichmäßig um die plane Anlagefläche (50) herum verteilt ist.

9. Spritze nach einem der Ansprüche 6 bis 8, wobei die vorspringenden Elemente (6) der Anordnung eine axiale Symmetrie in Bezug auf die Längsachse (Z'-Z) aufweisen.

10. Spritze nach einem der Ansprüche 1 bis 9, wobei die plane Anlagefläche (50) einen mit R bezeichneten Radius aufweist und das oder jedes vorspringende Element (6) ein seitliches Ende aufweist, das in einem mit r bezeichneten Abstand gelegen ist, für den gilt:
0,8 R ≤ r ≤ 1,20 R, bevorzugt R ≤ r ≤ 1,16 R.

11. Spritze nach einem der Ansprüche 1 bis 10, wobei das oder jedes vorspringende Element (6) und der Rastkopf (5) einstückig sind.

12. Spritze nach einem der Ansprüche 1 bis 11, wobei der Rastkopf (5) und das oder die vorspringenden Elemente (6) aus einem Kunststoff, bevorzugt einem Thermoplast, noch bevorzugter Polycarbonat, ausgeführt sind.

13. Spritze nach einem der Ansprüche 1 bis 12, wobei der Anschlag (2) aus einem Elastomermaterial, bevorzugt einem Chlor- oder Brombutylkautschuk, ausgeführt ist.

14. Spritze nach einem der Ansprüche 1 bis 13, wobei die Innenwand (20) des Anschlags (2) mindestens eine Schulter (21) umfasst, an der die plane Anlagefläche (50) des Rastkopfes (5) zur Anlage gelangt, wenn der Kolben entlang der zur Injektionsrichtung des Produkts entgegengesetzten Richtung gleitet; und das oder die vorspringenden Elemente (6) so gestaltet sind, dass sie in die Schulter (21) eindringen, wenn der Kolben entlang der zur Injektionsrichtung des Produkts entgegengesetzten Richtung gleitet.

## Claims

1. Syringe comprising:
- a syringe body (1) which is intended to receive a product for injection and which comprises a stopper (2); the stopper (2) having an elastically deformable inner wall (20) and delimiting a cavity (200);
- a piston which is mounted so as to be able to slide inside the syringe body (1) in an injection direction of the product and in an opposite direction and which comprises a rod (3) having a first end (30) and an opposite second end (31);
- a pusher (4) which is arranged at the first end (30) of the rod (3);
- a snap-fastening head (5) which extends along a longitudinal axis (Z'-Z), which is arranged at the second end (31) of the rod (3) and which is snap-fastened inside the cavity (200) of the stopper (2) by elastic engagement with the inner wall (20) of the stopper (2); the snap-fastening head (5) having a planar bearing surface (50); the planar bearing surface (50) is situated at a distance, denoted D, from the second end (31) of the rod (3); the planar bearing surface (50) defining a half-space extending towards the second end (31) of the rod (3);
the snap-fastening head (5) comprises at least one protruding element (6) which protrudes from the planar bearing surface (50) and which is arranged so as to penetrate the inner wall (20) of the stopper (2) when the piston slides in the direction opposite to the injection direction of the product, so as to anchor itself in the inner wall (20) of the stopper (2) by locally deforming said wall;
**characterized in that**:
- the planar bearing surface (50) bears against the inner wall (20) of the stopper (2) when the piston slides in the direction opposite to the injection direction of the product;
- the or each protruding element (6) extends in the half-space, and **in that** the or each protruding element (6) has a height, denoted H, along the normal to the planar bearing surface (50), such that 0.05 D ≤ H ≤ 0.2 D.

2. Syringe according to Claim 1, wherein the or each protruding element (6) comprises two planar surfaces (60, 61) forming an acute dihedral angle (α), preferably of between 10° and 80°, more preferably of between 30° and 60°, even more preferably of between 40° and 50°.

3. Syringe according to Claim 2, wherein the two planar surfaces (60, 61) of the or of each protruding element (6) are joined together so as to form an edge.

4. Syringe according to Claim 1, wherein the or each protruding element (6) comprises a conical or frustoconical surface exhibiting an acute cone angle, preferably of between 10° and 80°, more preferably of between 30° and 60°, even more preferably of between 40° and 50°.

5. Syringe according to Claim 1, wherein the or each protruding element (6) forms a tooth exhibiting an acute tip angle, preferably of between 10° and 80°, more preferably of between 30° and 60°, even more preferably of between 40° and 50°.

6. Syringe according to one of Claims 1 to 5, comprising a set of protruding elements (6) which protrude from the planar bearing surface (50) and which are arranged so as to penetrate the inner wall (20) of the stopper (2) when the piston slides in the direction opposite to the injection direction of the product.

7. Syringe according to Claim 6, wherein the protruding elements (6) of the set form patterns which are spaced apart periodically on the planar bearing surface (50).

8. Syringe according to Claim 6 or 7, wherein the planar bearing surface (50) is circular, and the set of protruding elements (6) is distributed uniformly around the planar bearing surface (50).

9. Syringe according to one of Claims 6 to 8, wherein the protruding elements (6) of the set exhibit axial symmetry with respect to the longitudinal axis (Z'-Z).

10. Syringe according to one of Claims 1 to 9, wherein the planar bearing surface (50) has a radius, denoted R, and the or each protruding element (6) has a lateral end situated at a radial distance, denoted r, such that:
0.8 R ≤ r ≤ 1.20 R, preferably R ≤ r ≤ 1.16 R.

11. Syringe according to one of Claims 1 to 10, wherein the or each protruding element (6) and the snap-fastening head (5) are in one piece.

12. Syringe according to one of Claims 1 to 11, wherein the snap-fastening head (5) and the protruding element or elements (6) are made of a plastics material, preferably a thermoplastic, more preferably polycarbonate.

13. Syringe according to one of Claims 1 to 12, wherein the stopper (2) is made of an elastomeric material, preferably a chlorinated or brominated butyl rubber.

14. Syringe according to one of Claims 1 to 13, wherein the inner wall (20) of the stopper (2) comprises at least one shoulder (21) against which the planar bearing surface (50) of the snap-fastening head (5) bears when the piston slides in the direction opposite to the injection direction of the product; and the protruding element or elements (6) are arranged so as to penetrate the shoulder (21) when the piston slides in the direction opposite to the injection direction of the product.
